(19) **Europäisches Patentamt**

**European Patent Office**

**Office européen des brevets**

(11) **EP 1 731 096 A1**

(12) **EUROPEAN PATENT APPLICATION**

(43) Date of publication:
**13.12.2006 Bulletin 2006/50**

(51) Int Cl.:
*A61B 5/107* (2006.01)  *A61F 13/08* (2006.01)
*A41H 1/02* (2006.01)

(21) Application number: **05012111.0**

(22) Date of filing: **06.06.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **Ganzoni Management AG**
**8400 Winterthur (CH)**

(72) Inventor: **Ganzoni, Stefan**
**4103 Bottmingen (CH)**

(74) Representative: **Blum, Rudolf Emil et al**
**E. Blum & Co**
**Vorderberg 11**
**8044 Zürich (CH)**

(54) **Devices and methods for characterizing medical compression garments**

(57) The problem to be solved by the present invention is to provide a means for improving the reliability and accuracy of the characterization of medical compression garments in wearing conditions. In a first aspect, this problem is solved by providing a device that comprises a means for determining a local radius of the limb's surface. This solution is based on the understanding that the tension in a medical compression garment is substantially constant while, according to Laplace's law, the local pressure P depends on the local radius of the limb's surface. The device can e.g. consist of a plurality of gauges (2), each of which has a circularly curved recess (3) and carries a label (6) indicative of a radius of said recess. The invention also relates to a method for characterizing a medical compression garment where a mark is applied to the garment worn on a limb. After removing the garment from the limb, the size and shape of the mark is reproduced in a dynamometer.

R = 20 mm
C = 125 mm

R = 50 mm
C = 314 mm

R = 80 mm
C = 502mm

**Fig. 1**

**Description**

The invention relates to devices and methods for characterizing medical compression garments or a pressure exerted by such compression garments.

Medical compression garments (abbreviated by MCG in the following text) are elastic garments for providing an interface pressure on the human skin, especially on a limb, such as an arm or a leg. MCGs can e.g. be used to compress a leg below the knee, a complete leg, an arm, a hand, etc., and they can be designed as stockings, socks, panties, arm sleeves, gloves, etc.

MCGs are classified by size and compression strength, see e.g. German norm RAL-GZ 387.

Such norms provide a global indicator for the properties of an MCG. It has been found, though, that such a global indicator can be insufficient for characterizing accurately the effect of a given MCG on a given limb.

To alleviate this problem, various tools for measuring the actual, local pressure exist. They are designed to be inserted between the limb and the MCG. It has been found, however, that these tools still yield results of poor reliability.

In particular in the context of medical studies, it is highly desirable to provide a means for characterizing the actual pressure exerted by an MCG.

Hence, the problem to be solved by the present invention is to provide a means for improving the reliability and accuracy of such characterizations.

In a first aspect of the invention, this problem is solved by providing a device that comprises a means for determining a local radius of the surface of the limb. This solution is based on the understanding that the tension T in an MCG is substantially constant while, according to Laplace's law, the local pressure P depends on the local radius of the surface of the limb. In good approximation the local pressure P is given by

$$P = T/R, \hspace{4cm} (1)$$

where R is the local radius of the limb's surface perpendicular to the limb's longitudinal axis. (Here, we assume that the local surface radius parallel to the limb's axis is much larger than the radius R perpendicular thereto, and the term "local radius" is used to designate the local radius of the limb surface perpendicular to the limb's longitudinal axis.)

Hence, a device of this type can be used to characterize the local effect of the MCG on the limb, as well as to characterize or evaluate an MCG. More details on various types of characterizations are given in the description below.

In one advantageous and simple embodiment, the device comprises a plurality of gauges, each gauge having a circularly curved recess and carrying a label indicative of a radius of said recess. Such a device is easy to manufacture and to use by applying the recess to the limb.

The label indicative of the radius of the recess can e.g. consist of an indication of the local radius R in mm and/or an indication of the corresponding circumference $C = 2\pi \cdot R$ and/or any code, figure, number, text or color coding that is uniquely correlated to these parameters.

In another embodiment, the device comprises two spaced apart contact probes that can be positioned against the limb and a position sensor for measuring a position of the limb's surface between the contact probes. Such a device relies on the fact that the local curvature or radius can be determined from three points on the surface and is suited for electronic detection. The position sensor may e.g. be an optical distance sensor with a measurement field between said contact probes.

In yet another embodiment, the device can comprise an image processor for processing a cross-sectional scan image of the limb and for calculating the local radius therefrom. This allows a precise, spatially resolved determination of the local radius and therefore of the local pressure that an MCG exerts on a limb.

In another aspect of the invention, a method is provided that allows to characterize an MCG. The method comprises the following steps:

donning the medical compression garment on a limb,
applying a mark having a given size to said compression garment or determining the size of a pre-applied mark while said compression garment is on said limb,
removing said compression garment from said limb, and
reproducing the given or determined size of the marking by exerting a tension on the compression garment and determining said tension.

In other words, the mark is applied to the stocking while it is in stretched state extended over the limb. Since the tension in the stocking is released when the stocking is removed from the limb, the size of the mark will change. By applying an extension to reproduce the size of the drawn pattern, the situation on the limb can be reproduced. The tension required to reproduce the size corresponds to the tension of the MCG on the limb.

The tension obtained in this way can e.g. be used per se, or it can be further processed e.g. by using Laplace's Law, i.e. equation (1) above, for calculating the local pressure P.

Further advantageous features and applications of the invention can be found in the dependent claims as well as in the following description thereof. The description refers to the following figures:

Fig. 1 is a top view of a first embodiment of the device according to the invention with only some of the gauges being visible,
Fig. 2 is a side view of the device of Fig. 1 with all gauges arranged on top of each other,
Fig. 3 shows a second embodiment of the device,
Fig. 4 is a top view of the second embodiment of the device while being applied to a limb,
Fig. 5 shows a third embodiment of the device,
Fig. 6 is a scan image to be analyzed by the device of Fig. 5,
Fig. 7 shows a stocking on a limb with a mark according to the second aspect of the invention, and
Fig. 8 shows the stocking of Fig. 7 in a measuring device.

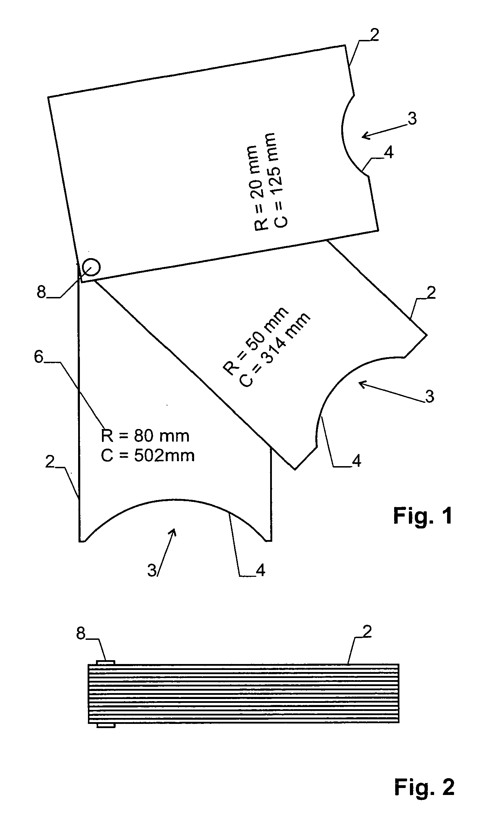

As mentioned above, one aspect of the present invention relates to a device that allows to measure the local curvature of a limb. An advantageous embodiment of such a device is shown in Figs. 1 and 2. It comprises a plurality of gauges 2, only three of which are shown in Fig. 1. Each gauge consists of a sheet-like material and has e.g. substantially rectangular shape. At one edge of each gauge 2, a circularly curved recess 3 is formed (i.e. a recess bordered by an edge 4 corresponding to a section of a circle).

Each gauge 2 has a recess 3 with a different radius, e.g. ranging from 20 to 80 mm with steps of 5 or 10 mm. The curvature of recess 3 is indicated, on each gauge, by a label 6. In the embodiment of Fig. 1, label 6 indicates the radius R as well as the circumference $C = 2\pi \cdot R$ of the corresponding circle, but label 6 may also display another parameter that allows the determine the radius R or circumference C.

The gauges 2 are advantageously self-supporting, i.e. they are somewhat stiff and do not significantly deform under normal gravity. They can e.g. be made from plastic, metal or cardboard (the term "cardboard" including firm paper).

The gauges 2 are connected to each other by means of a common carrier formed by a pin 8. Pin 8 extends through an opening in a corner of each gauge 2. The gauges 2 can be pivoted about pin 8 such that the recess 3 of each given gauge can be separated from the other gauges and be applied to a limb.

The device of Figs. 1 and 2 is used by placing the recess 3 of one gauge against a position of interest of the limb in such a way that the gauge stands perpendicular to the longitudinal axis of the limb. The curvature of recess 3 is then visually compared to the curvature of the limb. If the radius of the limb's surface is smaller, a gauge with a recess 3 of smaller radius is used and the measurement is repeated. Similarly, if the radius of the limb's surface is larger, a gauge with a recess 3 of larger radius is used and the measurement is repeated. The measurement ends once that the gauge with the best fitting recess 3 has been found. Label 6 of the corresponding gauge is then inspected to determine the curvature or radius.

Alternatively, the device can be used to find a place on a limb where it has a given radius. For that purpose, the gauge 2 corresponding to the given radius is slid along the limb horizontally of vertically until a place is found where it fits the local radius of the limb's surface.

The device of Figs. 1 and 2 should have gauges with recesses covering the range of curvatures found on a human limb. Typically, one device comprises, as mentioned above, gauges with recesses at least between 20 and 80 mm. However, for some applications devices with a more narrow range can be used as well.

The device of Figs. 1 and 2, or any other device that allows to determine the local curvature of a limb, can be used for various applications, such as:

a) Assuming that the tension T in the MCG is known, the pressure P at a point with local curvature R can be calculated from

$$P = T/R. \tag{2}$$

b) Similarly, assuming that the theoretical pressure $P_{R_0}$ (i.e. the pressure exerted by the given section of the MCG on a perfectly circular limb with radius $R_0$) is known, the pressure at a given position of the limb where the limb has the circumference $2\pi \cdot R_0$ and the local curvature R is given by

$$P = P_{R_0} \cdot R_0 / R. \qquad (3)$$

c) If the local pressure P at a position of curvature R where the limb has a circumference C is known (e.g. from a pressure sensor inserted at the given position), the theoretical pressure $P_{R_0}$ of the given section of the MCG on a circular limb with radius $R_0 = C/2\pi$ can be calculated from

$$P_{R_0} = P \cdot R / R_0, \qquad (4)$$

which allows to characterize the MCG.

d) If a given section on an MCG is specified to have a theoretical pressure $P_{R_0}$ on a circular limb with radius $R_0$, a measurement as indicated in c) can be carried out and equation (4) can be used to check if the MCG performs according to the specification.

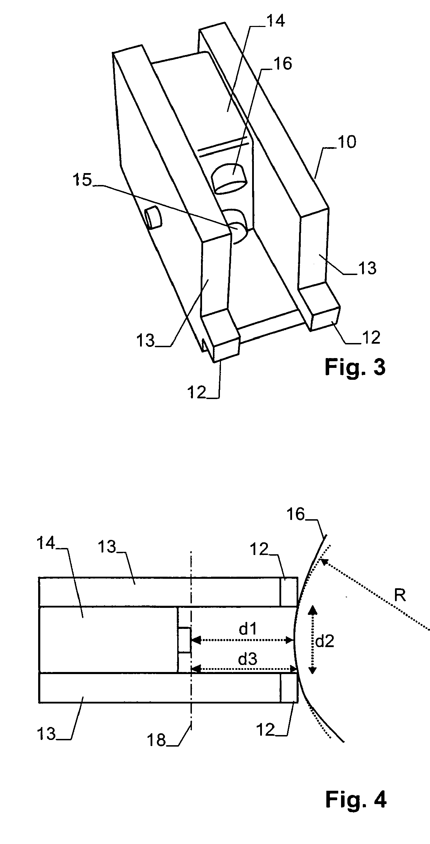

The device of Figs. 1 and 2 is only one of the possible embodiments of a device for measuring the local radius of the limb's surface. A second embodiment is shown in Fig. 3.

The second embodiment comprises a frame 10, which forms two spaced apart contact probes 12 to be placed against the limb. In the shown embodiment, the contact probes 12 are formed by two projections extending from two side plates 13 of housing 10. Furthermore, the device comprises a distance sensor 14 arranged between the side plates 13 for measuring the position of the limb's surface between the contact probes 12. Position sensor 14 is advantageously an optical distance sensor having a light emitter 15 emitting a bundled light beam along a measurement direction and an imaging optics with a position dependent light sensor 16 at an angle to the measurement direction. The device measures the position of the projected light spot on the light sensor and determines the distance therefrom. A corresponding device is e.g. offered by MEL Mikroelektronik GmbH, Eching (Germany). A calibration is of course necessary.

Fig. 4 shows how the device is applied to a limb by bringing the contact probes 12 into contact with the limb's surface 16. Position sensor 14 measures the distance d1 from a plane of reference 18 to the limb's surface 16 between the contact probes 12. Given the distance d2 between the position probes and the distance d3 of the position probes from the plane of reference 18, the local radius R can be calculated using known geometric dependencies.

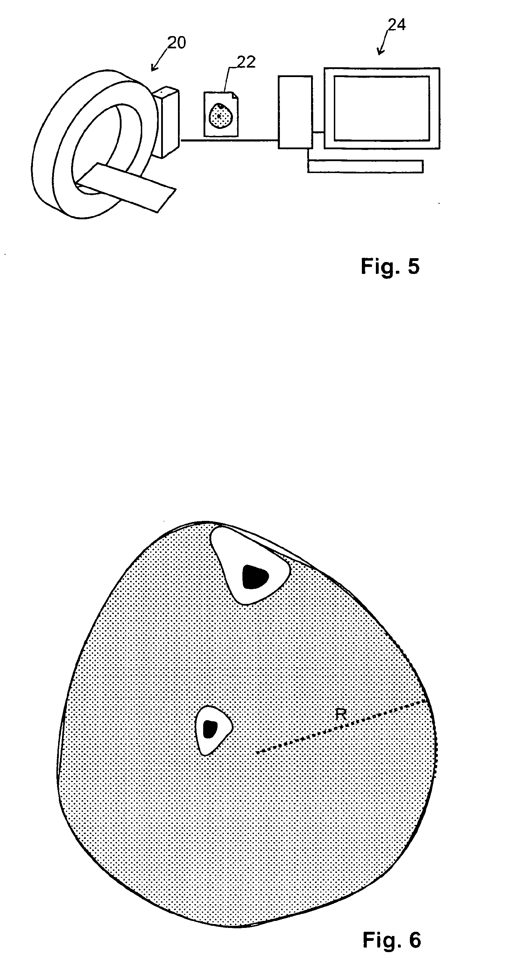

Yet another embodiment for measuring the local radius R of a limb is schematically shown in Fig. 5. It comprises a medical imaging device 20 for scanning a cross section of the limb. The medical imaging device 20 is capable to generate in non-destructive manner an image of the cross section of the limb. Such imaging devices or tomographs are e.g. based on one of the following technologies: NMR (nuclear magnetic resonance), ESR (electron spin resonance), PET (positron emission tomography).

Medical imaging device 20 generates a scanned cross-sectional image 22, as it is e.g. schematically shown in Fig. 6, which if fed to an image processor 24 e.g. implemented as a computer with suitable software. Image processor 24 processes the scanned cross-sectional image 22 of the limb and calculates the local radius R therefrom. The steps for processing the image 22 comprise, e.g.

1) determine the pixels occupied by the cross section of the limb by submitting the gray-level or color image 22 to a thresholding operation;
2) determine the convex hull of the occupied pixels by, e.g, a vectorization operation;
3) calculate the local curvature R along the convex hull or at certain points thereof;
4) given the tension T or the standard pressure $P_{R_0}$ of the MCG section at a radius corresponding to the circumference of the limb cross section, the local pressure P along the convex hull (i.e. along the circumference of the limb) can be calculated using equations (2) or (3).

This procedure allows to determine the local radius or pressure not only at a single point but at several points along the cross section in a single process. It even allows to calculate the pressure distribution over the whole limb if a sufficient number scanned images 22 is recorded. In addition, depending on the imaging technique that is used by medical imaging device 20, points of interest in the cross section (such as the location of varicose veins) may be identified directly, together with the local curvature and MCG pressure at these points.

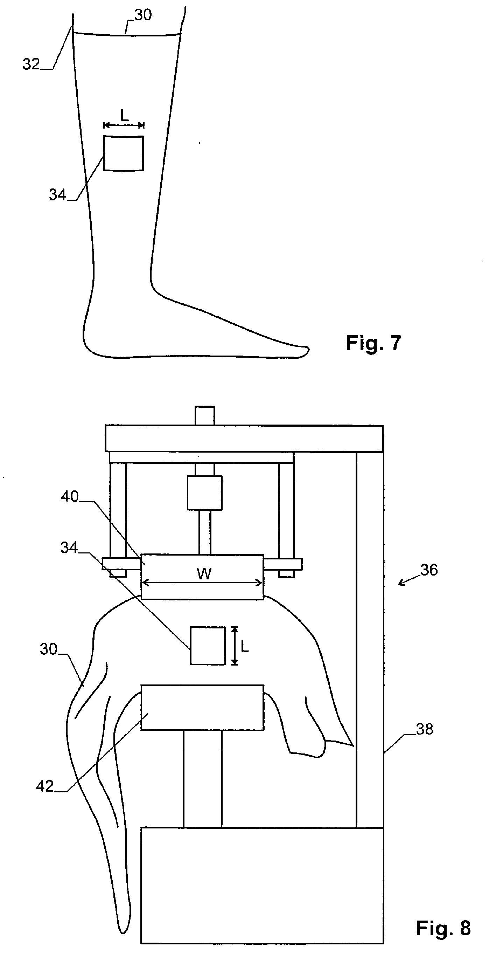

The second aspect of the invention is now explained with reference to Figs. 7 and 8. This second aspect of the invention allows to obtain an accurate characterization of an MCG's properties.

To characterize an MCG 30, it is first donned on a limb, as shown in Fig. 7. The limb 32 may be an artificial, e.g. standardized limb, but it may also be a natural limb.

Once the MCG 30 is in place, a mark 34 of defined size and shape is applied to it, advantageously by locally dying the MCG e.g. using paint and a stencil or by using a stamp. In the example of Fig. 7, mark 34 is a square having a given length L perpendicular to the longitudinal axis of the MCG.

Now MCG 30 is removed from limb 32, which will, in general, cause mark 34 to shrink. In an next step, MCG 30 is mounted to a dynamometer 36 as shown in Fig. 8. Dynamometer 36 comprises a frame 38, a movable clamp (including the force sensor) or jaw 40 and a static clamp or jaw 42. A corresponding device is e.g. disclosed in the "norm française NFG 30-102b". The MCG 30 is mounted such that a tension can be applied to mark 34 in the same direction as when worn on the limb 32. This means that, if mark 34 is applied to a substantially tubular section of MCG 30, the force must be applied in a direction perpendicular to "tube axis" of the tubular section.

The applied force is increased by increasing the distance between the jaws 40 and 42 until the size of mark 34 becomes the same as it was on limb 32. In the present example, this means that the extension is adjusted such that mark 34 again has a length L. A suitable force sensor is then used the measure the force.

The force F required for reconstructing a mark 34 of the same size as on the limb allows to calculate the tension T in the MCG using the formula

$$T = F/2W, \qquad\qquad (5)$$

where W is the width of the jaws 40. The factor 2 in equation (5) has to be used if MCG 30 has been mounted to dynamometer 36 in such a way that two layers of its tubular fabric are stretched simultaneously, and it must be replaced by 1 if only one layer is stretched.

The measurement of force F is preferably carried out at least five minutes after adjusting the device in order to obtain a measurement in stationary conditions (taking in consideration the material fatigue effect).

Once that tension T is known, the pressure P on limb 32 (or on any other limb having the same circumference at the given MCG section) can be calculated using equation (1).

The shape and size of mark 34 is not decisive. It must be large enough to allow reasonable measurements but it should be clearly smaller than the limb's half diameter. It may be a square, a grid, a simple line perpendicular to the MCGs longitudinal axis, or any other suitable shape. As follows from the above, only its extension L perpendicular to the MCGs longitudinal axis has to be known well.

In the example above, mark 34 was applied while MCG 30 was in a stretched state on limb 32. Alternatively, mark 34 can be applied prior to donning MCG 30 on limb 32 - in that case, the size (i.e. the length perpendicular to the MCG's longitudinal axis) of mark 34 must be determined e.g. by a measurement of length L while MCG 30 is stretched on limb 32.

As it becomes apparent from the above, the method of Figs. 7 and 8 allows to reconstruct the "conditions of wear" on the dynamometer accurately, which allows a precise measurement of the tension or pressure of the MCG.

The devices and methods described above can be e.g. used to characterize MCGs more accurately, for example during manufacturing or for standardization purposes. They can also be used in medical studies for assessing the compression parameters of MCGs with high accuracy, which allows to compare different experiments reliably.

## Claims

1.   A device for characterizing a pressure exerted by a medical compression garment on a limb, said device comprising means (2, 14, 24) for determining a local radius of a surface of said limb.

2.   The device of claim 1, wherein said device is adapted for determining a local radius in a range between at least 20 and 80 mm.

3.   The device of any of the preceding claims wherein said device comprises a plurality of gauges (2), each gauge having a circularly curved recess (3) and carrying a label (6) indicative of a radius of said recess.

4.   The device of claim 3 comprising a plurality of gauges (2) with recesses (3) having a radius between 20 and 80 mm.

5.   The device of any of the claims 3 or 4 wherein each gauge (2) comprises a sheet of plastic, metal or cardboard.

**6.** The device of any of the claims 3 to 5 wherein said gauges (2) are connected by a common carrier.

**7.** The device of claim 6 wherein said common carrier is a pin (8) extending through an opening in each gauge (2).

**8.** The device of any of the claims 6 or 7, wherein said gauges (2) are pivotal about said common carrier.

**9.** The device of claim 1 comprising two spaced apart contact probes (12) to be positioned against the limb and a position sensor (14) for measuring a position of the limb's surface between said contact probes, and in particular wherein said position sensor (14) is an optical distance sensor with a measurement field between said contact probes.

**10.** The device of claim 1 comprising an image processor (24) for processing a scanned cross-sectional image of the limb and calculating said local radius therefrom, and in particular wherein said image processor (24) is designed to calculate, from said local radius, a local pressure exerted by the medical compression garment on said limb

**11.** Use of the device of any of the preceding claims for characterizing or testing a medical compression garment.

**12.** A method for characterizing a medical compression garment comprising the steps of
donning the medical compression garment (30) on a limb (32),
applying a mark (34) having a given size to said compression garment (30) or determining a size of a pre-applied mark while said compression garment (30) is on said limb,
removing said compression garment (30) from said limb (32),
reproducing the given or determined size of the marking by exerting a tension on the compression garment and determining said tension.

**13.** The method of claim 12 wherein said mark is applied to a tubular section of said compression garment (32), wherein said known tension is generated by applying a force perpendicularly to a tubular axis of said tubular section.

**14.** The method of any of the claims 12 or 13 comprising the step of mounting said compression garment between two jaws (40, 42) of a measuring device (36) and increasing a distance between said jaws (40, 42) for generating said tension.

**15.** The method of any of the claims 12 to 14 comprising the step of locally applying dye to said compression garment (30) for applying said mark (34), and in particular wherein said dye is applied while said compression garment (30) is on said limb (32).

R = 20 mm
C = 125 mm

R = 50 mm
C = 314 mm

R = 80 mm
C = 502mm

Fig. 1

Fig. 2

**Fig. 3**

**Fig. 4**

**Fig. 5**

**Fig. 6**

**Fig. 7**

**Fig. 8**

**European Patent Office**

**EUROPEAN SEARCH REPORT**

Application Number

EP 05 01 2111

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 27 07 766 A (SALOMON GMBH) 24 August 1978 (1978-08-24) * page 4, line 20 - page 6, line 36 * * figures * | 1,2 | INV. A61B5/107 A61F13/08 A41H1/02 |
| X | DE 32 23 711 A (URLAUB) 5 January 1984 (1984-01-05) * page 6, line 4 - page 7, line 2 * * figure 3 * | 1 | |
| X | DE 37 05 718 A (FISCHBACH) 1 September 1988 (1988-09-01) * column 3, line 50 - column 5, line 8 * * figures 1-3 * | 1 | |
| A | DE 83 09 480 U (GERT LOUISODER GMBH & CO.) 22 March 1984 (1984-03-22) * the whole document * | 3-6,8 | |
| A | US 1 667 802 A (HOMAN) 1 May 1928 (1928-05-01) * the whole document * | 7 | TECHNICAL FIELDS SEARCHED (IPC) |
| X | US 6 338 723 B1 (CARPENTER ET AL.) 15 January 2002 (2002-01-15) * column 9, line 12 - column 13, line 22 * * column 18, line 7 - line 12 * * column 26, line 15 - column 27, line 51 * * figures 1-9,19,43-45 * | 12,13,15 | A61B A41H G01B A43D G01L G01N |

-/--

~~The present search report has been drawn up for all claims~~

| Place of search | Date of completion of the search | Examiner |
|---|---|---|
| The Hague | 20 April 2006 | Chen, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 05 01 2111

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | THOMAS ET AL.: "High-compression bandages" JOURNAL OF WOUND CARE, vol. 5, no. 1, 1996, pages 40-43, XP008062867 GB * page 41, right-hand column, line 20 - page 43, left-hand column, line 24 * | 12-15 | |
| X | US 6 334 363 B1 (TESTUD ET AL.) 1 January 2002 (2002-01-01) * column 2, line 62 - column 5, line 29 * * figures 1,2 * | 12,14 | |
| A | EP 1 118 851 A (FILODORO CALZE S.P.A.) 25 July 2001 (2001-07-25) * paragraph [0017] - paragraph [0039] * * paragraph [0054] - paragraph [0064] * * figures 1-4,6a-b * | 12-14 | |
| A | "MEASURING SUB-BANDAGE PRESSURE" JOURNAL OF WOUND CARE, MACMILLAN, LONDON, GB, vol. 9, no. 10, November 2000 (2000-11), pages 491-492, XP008062860 ISSN: 0969-0700 * abstract * | 12 | TECHNICAL FIELDS SEARCHED (IPC) |

~~The present search report has been drawn up for all claims~~

| Place of search | Date of completion of the search | Examiner |
|---|---|---|
| The Hague | 20 April 2006 | Chen, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................

& : member of the same patent family, corresponding document

EPO FORM 1503 03.82 (P04C01)

European Patent
Office

Application Number

EP 05 01 2111

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

12-15

☐ None of the further search fees have been paid within the fixed time limit. The present European search report has been drawn up for those parts of the European patent application which relate to the invention first mentioned in the claims, namely claims:

**European Patent Office**

**LACK OF UNITY OF INVENTION
SHEET B**

Application Number

EP 05 01 2111

The Search Division considers that the present European patent application does not comply with the requirements of unity of invention and relates to several inventions or groups of inventions, namely:

1. claims: 1-8, 11

   Plurality of gauges with circularly curved recesses for determining a local radius of a surface of a limb.
   ---

2. claims: 9-10

   Optical sensor for determinining a radius of a surface of a limb.
   ---

3. claims: 12-15

   Method of characterising a medical compression garment.
   ---

## ANNEX TO THE EUROPEAN SEARCH REPORT
## ON EUROPEAN PATENT APPLICATION NO.

EP 05 01 2111

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2006

| Patent document cited in search report | | Publication date | Patent family member(s) | | Publication date |
|---|---|---|---|---|---|
| DE 2707766 | A | 24-08-1978 | NONE | | |
| DE 3223711 | A | 05-01-1984 | NONE | | |
| DE 3705718 | A | 01-09-1988 | NONE | | |
| DE 8309480 | U | 22-03-1984 | NONE | | |
| US 1667802 | A | 01-05-1928 | NONE | | |
| US 6338723 | B1 | 15-01-2002 | NONE | | |
| US 6334363 | B1 | 01-01-2002 | AT | 284188 T | 15-12-2004 |
| | | | CA | 2295842 A1 | 30-12-1998 |
| | | | DE | 69828053 D1 | 13-01-2005 |
| | | | DE | 69828053 T2 | 13-04-2006 |
| | | | EP | 0993283 A1 | 19-04-2000 |
| | | | ES | 2235342 T3 | 01-07-2005 |
| | | | FR | 2764796 A1 | 24-12-1998 |
| | | | WO | 9858605 A1 | 30-12-1998 |
| | | | JP | 2002510391 T | 02-04-2002 |
| | | | PT | 993283 T | 29-04-2005 |
| EP 1118851 | A | 25-07-2001 | IT | FI20000008 A1 | 23-07-2001 |

EPO FORM P0459

For more details about this annex : see Official Journal of the European Patent Office, No. 12/82